# EUROPÄISCHE PATENTSCHRIFT

(11) **EP 1 056 682 B1**
(45) Veröffentlichungstag und Bekanntmachung des Hinweises auf die Patenterteilung: **25.09.2002**
(21) Anmeldenummer: 99913205.3
(22) Anmeldetag: 01.03.1999
(51) Int. Cl.: C01B 15/023

(54) **VERFAHREN ZUR SUSPENSIONSHYDRIERUNG EINER ANTHRACHINON-VERBINDUNG IN EINEM SPEZIELLEN REAKTOR ZUR HERSTELLUNG VON WASSERSTOFFPEROXID**
METHOD FOR SUSPENSION HYDROGENATION OF AN ANTHRAQUINONE COMPOUND IN A SPECIAL REACTOR IN ORDER TO PRODUCE HYDROGEN PEROXIDE
PROCEDE D'HYDROGENATION EN SUSPENSION D'UN COMPOSE ANTHRAQUINONE DANS UN REACTEUR SPECIFIQUE POUR PREPARER DU PEROXYDE D'HYDROGENE

(30) Priorität: 27.02.1998 DE 19808385
(43) Veröffentlichungstag der Anmeldung: 06.12.2000
(73) Patentinhaber: BASF AKTIENGESELLSCHAFT, 67056 Ludwigshafen (DE)
(72) Erfinder: Dr. BOETTCHER, Arnd, D-67227 Frankenthal (DE); HENKELMANN, Jochem, D-68165 Mannheim (DE); BROECKER, Franz, Josef, D-67061 Ludwigshafen (DE); KAIBEL, Gerd, D-68623 Lampertheim (DE); RUETTER, Heinz, D-67126 Hochdorf-Assenheim (DE)
(74) Vertreter: Isenbruck, Günter, Dr.
(86) Internationale Anmeldenummer: EP9901324
(87) Internationale Veröffentlichungsnummer: WO99043611

(56) Entgegenhaltungen:
- EP-A- 0 672 617
- DE-A- 19 611 976
- US-A- 4 428 923

## Beschreibung

Die vorliegende Erfindung betrifft ein Verfahren zur Suspensionshydrierung von Anthrachinon-Verbindungen in einem speziellen Reaktor zur Herstellung von Wasserstoffperoxid nach dem Anthrachinonverfahren. Bei dem erfindungsgemäßen Hydrierverfahren wird eine Anthrachinon-Verbindung oder ein Gemisch aus zwei oder mehr davon mit einem Suspensionskatalysator und einer Wasserstoff enthaltenden Gasphase in einem speziellen Reaktor, wie er in der DE-A 196 11 976 ausführlich beschrieben wird, in Kontakt gebracht. Dieser spezielle. Reaktor enthält Vorrichtungen mit Öffnungen oder Kanälen, die einen bestimmten hydraulischen Durchmesser besitzen.

Nach dem sogenannten Anthrachinon-Verfahren wird praktisch die gesamte Menge des weltweit produzierten Wasserstoffperoxids (> 2 Mio t/a) hergestellt.

Das Verfahren beruht auf der katalytischen Hydrierung einer Anthrachinon-Verbindung zur entsprechenden Anthrahydrochinon-Verbindung, nachfolgender Umsetzung derselben mit Sauerstoff unter Bildung von Wasserstoffperoxid und anschließender Abtrennung des gebildeten Wasserstoffperoxids durch Extraktion. Der Katalysezyklus wird durch erneute Hydrierung der rückgebildeten Anthrachinon-Verbindung geschlossen.

Einen Überblick über die prinzipiellen Reaktionsabläufe gibt das unten dargestellte Schema:

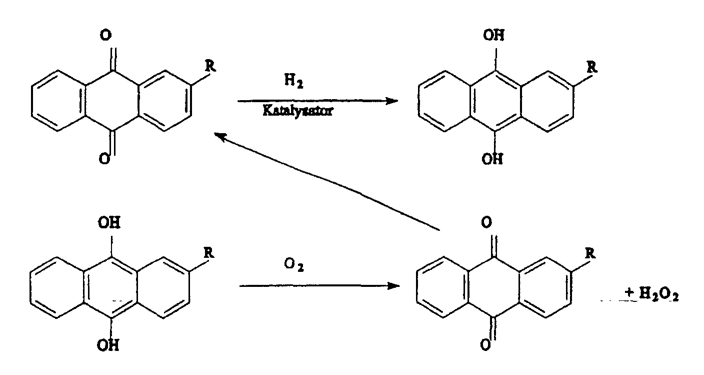

Dabei werden die verwendeten Anthrachinon-Verbindungen in der Regel in einem Gemisch mehrerer organischer Lösungsmittel gelöst. Die resultierende Lösung wird als Arbeitslösung bezeichnet. Diese Arbeitslösung wird beim Anthrachinon-Verfahren in der Regel kontinuierlich durch die oben beschriebenen Stufen des Verfahrens geführt.

Einen Überblick über das Anthrachinon-Verfahren gibt Ullmanns Encyclopedia of Industrial Chemistry, 5. Aufl., Bd. A13, S. 447-456.

Eine besonders wichtige Stufe des Anthrachinon-Verfahrens stellt die Hydrierstufe dar, in der die in der Arbeitslösung enthaltene Anthrachinon-Verbindung in Gegenwart eines Katalysator zur entsprechenden Anthrahydrochinon-Verbindung hydriert wird.

Die vorliegende Erfindung betrifft diese Hydrierstufe des Anthrachinonverfahrens.

Diese katalytische Hydrierung kann in Suspension oder im Festbett in unterschiedlichen Reaktortypen erfolgen. Der Stand der Technik wird z.B. ausführlich in EP 0 672 617 gewürdigt.

In einem Festbettverfahren werden die Wasserstoff enthaltende Gasphase und die Arbeitslösung im Gleich- und Gegenstrom durch einen Reaktor geleitet, der mit einem Edelmetall-belegten, geträgerten Katalysator gefüllt ist. Der verwendete Katalysator verliert im Laufe der Zeit an Aktivität und muß daher regeneriert oder ersetzt werden. Hierfür muß der Festbettkatalysator zunächst aus dem Reaktor ausgebaut werden und neuer bzw. regenerierter Katalysator eingebaut werden. Dies ist sehr zeitraubend und teuer.

Die Hydrierstufe wird daher industriell vornehmlich in Suspensionsfahrweise durchgeführt, da einem Nachlassen der Aktivität des Katalysators durch kontinuierliches Ein- und Ausschleusen desselben begegnet werden kann.

Die Suspensionshydrierung erfolgt in ihrer allgemeinsten Form in einem Reaktor, der die Arbeitslösung enthält, in der mindestens ein Katalysator suspendiert ist und der zusätzlich eine Wasserstoff enthaltende Gasphase enthält.

Die Technologie für Suspensionsreaktoren allgemein ist in Ullmanns Enzyklopädie der technischen Chemie, 4. Auflage, Band 3, S. 494- 518, ausführlich beschrieben.

Einige Reaktoren, die für die Suspensionshydrierung von Anthrachinon-Verbindungen eingesetzt werden sind in Ullmanns Enzyklopädie der technischen Chemie, 4. Auflage, Band 17, S. 700- 702 beschrieben. Hierunter befinden sich Rührkessel-, Blasensäulen und Fließbettreaktoren.

Als Katalysatoren für die Suspensionshydrierung von Anthrachinon-Verbindungen werden entweder Suspensionskatalysatoren oder Trägersuspensionskatalysatoren eingesetzt. Bei den letzteren befindet sich eine Metallschicht auf einem Trägerteilchen. Trägersuspensionskatalysatoren haben den Vorteil, daß die Teilchendurchmesser von 0,06 bis 0,15 mm die Rückführung des Katalysators in den Reaktor gegenüber nicht-geträgerten Suspensionskatalysatoren vereinfachen. Außerdem sind sie im allgemeinen hinsichtlich ihrer Aktivität unempfindlicher gegen thermische Belastung und Gifteinwirkung als reine Katalysatoren.

Die Suspensionshydrierung einer Anthrachinon-Verbindung unter Verwendung eines Schlaufenreaktors mit Palladium-Mohr als Katalysator wird in der US 4 428 923 beschrieben.

In der DE-PS 938 252 erfolgt die Hydrierung der Anthrachinon-Verbindung in einer Blasensäule, die Rohreinbauten enthält, in welchen es durch die Einleitung von Wasserstoff in den unteren Teil eines jeden Rohres zu einer Aufwärtsströmung der Arbeitslösung in den Rohren kommt. Als Katalysator wird ein Palladium-Trägersuspensionskatalysator (z.B. 2% Pd auf aktivem Aluminiumoxid) eingesetzt.

Grundproblem von Suspensionsreaktionen ist die ausreichende Kontaktierung der Reaktionsedukte durch die Katalysatorpartikel, die in der Flüssigphase suspendiert sind.

Suspensionsreaktoren benötigen die Zufuhr mechanischer Energie, die beispielsweise durch Rührwerke, Düsen oder aufsteigende Gasblasen eingebracht wird, um die Feststoffteilchen zu suspendieren. Eine Erhöhung dieser mechanischen Energiezufuhr über den zur Suspendierung erforderlichen Betrag hinaus, führt aber zu keiner nennenswerten Verbesserung des Stoffübergangs zwischen der Flüssigkeit und den suspendierten Feststoffteilchen, da die erzielbare Relativgeschwindigkeit die Sedimentationsgeschwindigkeit nur unwesentlich übertrifft.

Entscheidend für das wirtschaftliche Betreiben eines Anthrachinonverfahrens ist eine hohe Raum-Zeit-Ausbeute in der Hydrierstufe.

Die Raum-Zeit-Ausbeute ist die auf die Einheit des Katalysatorvolumens und die Zeiteinheit bezogene Menge an gebildetem Produkt.

Mit den bislang gemäß des Standes der Technik verwendeten Reaktoren für die Suspensionshydrierung von Anthrachinon-Verbindungen zur Herstellung von Wasserstoffperoxid war es nicht immer möglich, ausreichend hohe Raum-Zeit-Ausbeuten zu erreichen.

Somit ist es die Aufgabe der vorliegenden Erfindung, ein Verfahren zur Suspensionshydrierung einer Anthrachinon-Verbindung unter Verwendung eines bislang noch nicht für diese Hydrierung eingesetzten Reaktors bereitzustellen.

Diese Aufgabe wird durch die das in den Ansprüchen beschriebene Verfahren gelöst. Bei diesem Verfahren zur Durchführung der Suspensionshydrierung einer Anthrachinon-Verbindungen oder eines Gemisches aus zwei oder mehr davon in einem Reaktor, der die Arbeitslösung enthält, in der mindestens ein Katalysator suspendiert ist, und der zusätzlich eine Wasserstoff enthaltende Gasphase enthält, werden die Arbeitslösung und die Gasphase zumindest teilweise, d.h. mit einem Teil ihres Volumen der auf einem Teil ihres Weges durch eine Vorrichtung mit Öffnungen oder Kanälen in dem Reaktor geführt, deren hydraulische Durchmesser 0,5 bis 20 mm, vorzugsweise 1 bis 10 mm, besonders bevorzugt 1 bis 3 mm, beträgt. Der hydraulische Durchmesser ist als Quotient des 4fachen Querschnitts der Öffnung und deren Umfang definiert.

Die Wahl der im Einzelfall idealen Kanalbreite hängt vor allem von der Viskosität der hindurchgeleiteten Flüssigkeit, der Größe der suspendierten Teilchen und der Art der Gasphase ab. Die Kanalbreiten müssen umso größer sein, je viskoser die Flüssigkeit ist. Bei Flüssigkeiten mit dynamischen Viskositäten zwischen 10 x 10⁻⁵ und 200 x 10⁻⁵ Ns/m² sind hydraulische Durchmesser im Bereich von 1 bis 4,5 mm optimal.

Hierbei werden höhere Raum-Zeit-Ausbeuten als in konventionellen Reaktoren zur Suspensionshydrierung von Anthrachinon-Verbindungen erhalten.

Im Rahmen des erfindungsgemäßen Verfahrens wird die Hydrierstufe im allgemeinen bei einer Temperatur von ungefähr 20 bis 120 °C, vorzugsweise ungefähr 30 bis 80 °C, durchgeführt. Die dabei verwendeten Drücke liegen in der Regel bei ungefähr 1 bis ungefähr 20 bar, vorzugsweise ungefähr 2 bis 10 bar.

Zur Hydrierung kann entweder reiner Wasserstoff oder ein Wasserstoff enthaltendes Gas verwendet werden.

Dabei wird im allgemeinen die Hydrierung bis zu einem Umsatz von ungefähr 50 bis 70 % durchgeführt, um eine möglichst hohe Selektivität von im allgemeinen > 90%, vorzugsweise > 95% zu erreichen.

Der Begriff "Anthrachinon-Verbindung" umfaßt prinzipiell alle Anthrachinon-Verbindungen sowie die entsprechenden Tetrahydroanthrachinon-Verbindungen, die für das Anthrachinon-Verfahren zur Herstellung von Wasserstoffperoxid eingesetzt werden können. Anthrachinon-Verbindungen die bevorzugt für das erfindungsgemäß Verfahren eingesetzt werden sind 2-Alkylanthrachinone, wie z.B. 2-Ethyl-, 2-tert.-Butyl, 2-Amyl-, 2-Methyl-, 2-Butyl-, 2-Isopropyl-, 2-sec.-Butyl-, 2-sec.-Amylanthrachinon, sowie Polyalkylanthrachinone, wie z.B. 1,3-Diethylanthrachinon, 2,3-Dimethylanthrachinon, 1,4-Dimethylanthrachinon, 2,7-Dimethylanthrachinon, sowie die entsprechenden Tetrahydroanthrachinon-Verbindungen, sowie Gemische aus zwei oder mehr davon eingesetzt.

Als Lösungsmittel lassen sich alle aus dem Stand der Technik bekannten Lösungsmittel für Anthrachinon- bzw. Anthrahydrochinon-Verbindungen einsetzen. Dabei sind Gemische aus zwei oder mehr Lösungsmittelkomponenten bevorzugt, da derartige Lösungsmittelgemische am ehesten den unterschiedlichen Lösungseigenschaften von Anthrachinon- und Anthrahydrochinon-Verbindung Rechnung tragen. Beispielhaft zu nennen sind u.a. Gemische aus Methylnaphthalin und Nonylalkohol, Methylnaphthalin und Tetrabutylharnstoff, polyalkyliertem Benzol und Alkylphosphaten oder Methylnaphthalin, Tetrabutylharnstoff und Alkylphosphaten zu nennen.

Als Katalysatoren können alle aus dem Stand der Technik bekannten Katalysatoren eingesetzt werden, die für Suspensionsfahrweise geeignet sind, wie z.B. Raney-Nickel oder Pd-Mohr. Man unterscheidet zwischen nicht-geträgerte Katalysatoren und geträgerten Katalysatoren. Als Katalysatoren kommen Metalle, vorzugsweise Edelmetalle zum Einsatz. Nicht-geträgerte Katalysatoren, die im Rahmen des erfindungsgemäßen Verfahrens eingesetzt werden können, sind alle Metalle der VIII. Nebengruppe des Periodensystems. Vorzugsweise werden Platin, Rhodium, Palladium, Cobalt, Nickel oder Ruthenium oder ein Gemisch aus zwei oder mehr davon eingesetzt, wobei insbesondere Ruthenium als Katalysator verwendet werden kann. Unter den ebenfalls als nicht-geträgerte Katalysatoren verwendbaren Metallen der 1. und VII. Nebengruppe des Periodensystems, die ebenfalls allesamt prinzipiell verwendbar sind, werden vorzugsweise Kupfer und/oder Rhenium eingesetzt. Des weiteren können Metallsalze bzw. -oxide, wie z.B. Rheniumsulfide, Kupferchromide, Zinkchromide. Nickeloxide, Molybdänoxide, Aluminiumoxide, Rheniumoxide und Zinkoxide als Katalysatoren im Rahmen des erfindungsgemäßen Verfahrens eingesetzt werden.

Bevorzugt werden Trägersuspensionskatalysatoren eingesetzt. Diese bestehen aus Trägerpartikeln die mit Metallen, bevorzugt Edelmetallen, beschichtet sind. Aktivmetalle, die für solche Kontakte eingesetzt werden können sind prinzipiell alle Metalle der VIII. Nebengruppe des Periodensystems. Vorzugsweise werden als Aktivmetalle Platin, Rhodium, Palladium. Cobalt, Nickel oder Ruthenium oder ein Gemisch aus zwei oder mehr davon eingesetzt, wobei insbesondere Ruthenium als Aktivmetall verwendet wird. Unter den ebenfalls verwendbaren Metallen der I. und VII. Nebengruppe des Periodensystems. die ebenfalls allesamt prinzipiell verwendbar sind, werden vorzugsweise Kupfer und/oder Rhenium eingesetzt.

Obwohl prinzipiell alle bei der Katalysatorherstellung bekannten Trägermaterialien eingesetzt werden können, werden vorzugsweise Aktivkohle, Siliciumcarbid, Aluminiumoxid, Siliciumoxid, Siliciumdioxid, Titandioxid, Zirkoniumdioxid, Magnesiumoxid, Zinkoxid, Calciumcarbonat, Bariumsulfat oder deren Gemische, weiter bevorzugt Aluminiumoxid und Zirkoniumdioxid, eingesetzt.

Der Gehalt des Aktivmetalls beträgt bei den verwendeten Trägersuspensionskatalysatoren im allgemeinen ungefähr 0,01 bis ungefähr 30 Gew.%, vorzugsweise ungefähr 0,01 bis ungefähr 5 Gew.% und insbesondere ungefähr 0,1 bis 5 Gew.% jeweils bezogen auf das Gesamtgewicht des verwendeten Katalysator.

Bei dem erfindungsgemäßen Verfahren führen die Katalysatorteilchen gegenüber der Flüssigphase eine verstärkte Relativbewegung aus, weil sie in den engen Öffnungen und Kanälen eine Abbremsung gegenüber der umgebenden Arbeitslösung erfahren. Diese Abbremsung kann durch Kollisionen mit den Kanalwandungen ebenso verursacht werden wie durch kurzes Festhalten der Teilchen an rauhen Wandflächen.

Für das erfindungsgemäßen Verfahren können suspendierte Katalysatorpartikel mit einer mittleren Korngröße von 0,0001 bis 2 mm, besonders bevorzugt von 0,001 bis 0,1 mm, weiter bevorzugt von 0,005 bis 0,05 mm, verwendet werden. Diese Partikel führen mit ihrer hohen volumenbezogenen Oberfläche zu guten Resultaten, denn sie können infolge der Durchleitung durch die engen Einbauten Relativbewegungen gegenüber der Arbeitslösung ausführen. Im Ergebnis können deutlich höhere Raum-Zeit-Ausbeuten erzielt werden. Dabei zeigte sich im Versuch, daß bereits geringe Relativbewegungen der Katalysatorteilchen bzw. eine Abbremsung eines nur kleinen Teils der Katalysatorteilchen zu einer Beschleunigung der Reaktion führen.

Die Vorrichtung mit Öffnungen oder Kanälen innerhalb des Reaktors zur Durchführung der Eduktphase kann in einer Schüttung, einem Gestrick, einer offenzelligen Schaumstruktur, vorzugsweise aus Kunststoff (z.B. Polyurethan oder Melaminharze) oder Keramik, oder einem Packungselement, wie es grundsätzlich, d.h. seiner geometrischen Form nach, bereits aus der Destillations- oder Extraktionstechnik bekannt ist, bestehen. Derartige Pakungselemente, die den Vorteil eines geringen Druckverlusts bieten, sind zum Beispiel Drahtgewebepackungen der Bauart Montz A3 und Sulzer BX, DX und EX. Für die Durchführung des erfindungsgemäßen Verfahrens haben die Packungen jedoch grundsätzlich einen wesentlich, regelmäßig um den Faktor 2 bis 10 kleineren hydraulischen Durchmesser als vergleichbare Einbauten im Bereich der Destillations- oder Extraktionstechnik. Drahtgewebepackungen sind besonders vorteilhaft. Das beruht vermutlich darauf, daß ein Teil der Suspension nicht den gebildeten Kanälen folgt, sondern durch das Gewebe hindurchtritt. Statt Gewebepackungen können aber im Rahmen des erfindungsgemäßen Verfahrens auch Packungen aus anderen gewebten, gewirkten oder gefilzten flüssigkeitsdurchlässigen Materialien verwendet werden. Bei weiteren geeigneten Packungen werden ebene Bleche, bevorzugt ohne Perforationen oder andere größere Öffnungen, eingesetzt, beispielsweise entsprechend den Bauarten Montz B1 oder Sulzer Mellapak. Vorteilhaft sind auch Packungen aus Streckmetall, wie zum Beispiel Packungen des Typs Montz BSH. Auch dabei müssen Öffnungen, wie etwa Perforationen, entsprechend klein gehalten werden. Entscheidend für die Eignung einer Packung im Rahmen der vorliegenden Erfindung ist nicht deren Geometrie, sondern die für die Stromführung entstehenden Öffnungsgrößen bzw. Kanalbreiten in der Packung.

Bevorzugt wird bei dem erfindungsgemäßen Verfahren die Arbeitslösung und die Gasphase durch Öffnungen oder Kanäle geführt, deren Wandmaterialien Oberflächenrauhigkeiten im Bereich des 0,1- bis 10fachen. vorzugsweise des 0,5- bis 5fachen, der mittleren Korngröße der suspendierten Katalysatorteilchen aufweisen. Die Aufrauhung der Öffnungen bzw. Kanalwände bewirkt eine besonders gute Abbremsung und damit Relativbewegung der suspendierten Katalysatorteilchen. Diese werden an den Wandflächen vermutlich kurz festgehalten, so daß sie erst verzögert wieder in den Flüssigkeitsstrom eintreten. Dabei hängt die bevorzugte Rauhigkeit des verwendeten Materials im Einzelfall von der Größe der suspendierten Katalysatorpartikel ab.

Ferner werden bei dem erfindungsgemäßen Verfahren die Arbeitslösung und die Wasserstoff enthaltende Gasphase bevorzugt durch Öffnungen oder Kanäle mit metallischen Wandmaterialien geführt, deren Oberflächen einen Mittenrauhwert Rₐ nach DIN 4768/1 von 0,001 bis 0,01 mm aufweisen. Solche metallischen Oberflächen können zum Beispiel durch thermische Behandlung von Stählen, zum Beispiel Kanthal (Werkstoff-Nr.: 1.4767), in Sauerstoffatmosphäre hergestellt werden. Wirksam im Sinne der vorliegenden Erfindung sind somit nicht nur makroskopische, sondern auch mikroskopische Rauhigkeiten.

Bevorzugt wird das erfindungsgemäße Verfahren so durchgeführt, daß die Flüssigphase mit einer Leerrohrgeschwindigkeit von etwa 50 bis 300 m³/m²h, vorzugsweise von 150 bis 200 m³/m²h, durch die Vorrichtung mit Öffnungen und Kanälen geführt wird. Die Leerrohrgeschwindigkeit der Gasphase beträgt vorzugsweise 0,15 bis 8,5 cm/s, besonders bevorzugt 2,5 bis 5,5 cm/s.

Das erfindungsgemäße Verfahren kann kontinuierlich oder diskontinuierlich durchgeführt werden.

Der Reaktor in welchen erfindungsgemäß die Hydrierung der Anthrachinon-Verbindungen erfolgt, umfaßt alle aus dem Stand der Technik für Suspensionsreaktionen bekannten Reaktorbauformen wie z.B. Strahldüsenreaktoren, Blasensäulen, Fließbettreaktoren, Rohrreaktoren, Rohrbündelreaktoren und Rührbehälter. Bei einem Rührbehälter können die oben beschriebenen Einbauten auch direkt auf der Rührerwelle befestigt sein und zumindest teilweise die Funktion eines Rührorgans übernehmen. Sie können auch zusätzlich als Strombrecher wirken. Die oben vorgestellten Einbauten füllen - abgesehen von der Anwendung in Rührbehältern - vorzugsweise, aber nicht notwendigerweise den gesamten Reaktor aus. Der Reaktor ist vorzugsweise eine vertikal angeordnete Blasensäule, die bevorzugt im Gleichstrom von unten nach oben durchströmt wird. Eine andere bevorzugte Reaktorbauform ist ein heiz- oder kühlbarer Rohrbündelreaktor, bei dem die Einbauten in den einzelnen Rohren untergebracht sind. Dieser Reaktor wird vorzugsweise im Gleichstrom von unten nach oben durchströmt. Geeignet ist ebenso ein Rührbehälter, bei dem die Einbauten in die Strombrecher und/oder Rührorgane integriert sind.

Der Reaktor verfügt über Einrichtungen zur Zu- und Abführung der Arbeitslösung und der Gasphase.

Das suspendierte Katalysatormaterial kann mit Hilfe gebräuchlicher Techniken eingebracht und wieder abgetrennt werden (Sedimentation, Zentrifugation, Kuchenfiltration, Querstromfiltration).

Die Abtrennung der suspendierten Feststoffe erfolgt nach den üblichen Trennungsverfahren, wie zum Beispiel Kuchenfiltration oder Kerzenfiltration. Als besonders geeignet erwies sich bei kontinuierlicher Reaktionsführung die Querstromfiltration.

Die vorliegende Erfindung wird folgend im Detail beschrieben.

Beispielhaft ist ein Versuchsaufbau mit einem diskontinuierlich betriebenen Blasensäulenreaktor, in dem entsprechend der vorliegenden Erfindung eine Gewebepackung angeordnet ist, deren Geometrie mit der Destillationspackung Sulzer BX vergleichbar ist. Zur Durchführung der Reaktion wird zunächst die Arbeitslösung mit suspendiertem Katalysator über eine Befülleitung eingebracht. Über eine Anschlußleitung wird die Wasserstoff enthaltende Gasphase zugefahren und in einer Mischdüse mit der umgepumpten Suspension vermischt und dieses Gemisch am unteren Ende des Reaktors zugefahren. Die Suspension wird über eine erste Leitung zusammen mit Gas aus dem Reaktor ausgetragen und in ein Abscheidegefäß geleitet. Von dort wird das Gas über einen Abgaskühler geleitet und über eine Druckhaltung in eine Abgasleitung geführt. Die Suspension gelangt aus dem Abscheidegefäß über eine zweite Leitung in eine Pumpe, einen Wärmetauscher, eine Mischdüse und weiter in den Reaktor. Nach beendeter Reaktion wird die Suspension über eine Entnahmeleitung abgelassen.

Weiterhin beispielhaft ist eine kontinuierlich betriebene Blasensäule mit Packungen, die über eine dritte und vierte Leitung zusätzlich mit Kreisgas betrieben wird, das zusammen mit einem Wasserstoff enthaltenden Frischgas über die Mischdüse in die im Kreis geführte Suspension eingemischt wird. Der Reaktoraustrag wird über die erste Leitung in das Abscheidegefäß gefahren, in dem die Gasphase abgeschieden und über die dritte Leitung abgeführt wird. Von dieser Gasmenge wird zur Begrenzung der Aufpegelung von gasförmigen Verunreinigungen ein Teilstrom über eine fünfte Leitung entnommen und die verbleibende Restmenge über die vierte Leitung wieder in den Reaktor geführt. Über die Befülleitung wird nur flüssige Arbeitslösung eingebracht. Der suspendierte Katalysator verbleibt in dem Reaktorsystem, indem er über ein Querstromfilter zurückgehalten wird und nur katalysatorfreie Flüssigkeit austritt und entnommen wird.

Des weiteren beispielhaft ist eine bei schnell verlaufenden Reaktionen mit hoher Wärmetönung bevorzugt einzusetzende Ausführungsform, bei der der Reaktor die Bauform eines Rohrbündelwärmetauschers aufweist und Rohrbündel besitzt, in denen Drahtgewebefüllungen angeordnet sind. Der zuvor beschriebene Wärmetauscher kann bei dieser Ausführungsform entfallen. Er kann beibehalten werden, um die Funktion eines Aufheizers zu Beginn der Reaktion zu übernehmen, wenn der Reaktor nur mit Kühlmittel beaufschlagt werden soll. Die Funktionen der übrigen Teile entsprechen denen der zuvor genannten.

Die in der DE-A 196 11 976 beschriebenen weiteren Einzelheiten zum Reaktor, zur Verfahrensführung, zum Versuchsaufbau, zum verwendeten Katalysator und zu den verwendeten Packungselementen kommen im Rahmen des erfindungsgemäßen Verfahrens sinngemäß zur Anwendung.

Die vorliegende Erfindung soll nunmehr anhand von folgendem Beispiel näher erläutert werden:

### BEISPIEL

Die diskontinuierliche Hydrierung von 2-Ethylanthrachinon (13%ige Lösung in Shellsol / Tetrabutylharnstoff(70:30)) wurde einerseits in einem herkömmlichen 1 1-Rührbehälter mit dreiflügeligem Begasungsrührer (1000 U/min), andererseits in einer mit einer Gewebepackung bestückten Blasensäule (400 mm Höhe, 40 mm Durchmesser) bei 30 °C und Umgebungsdruck durchgeführt. Als Suspensionskatalysator wurden 1.5 g Pd auf Aluminiumoxid (Pd-Gehalt: 5 % ) verwendet. Die Menge an eingesetzter Arbeitslösung betrug in beiden Fällen 650 ml.
a) Rührbehälter: Es wurde insgesamt 3 h hydriert. Nach 3 h war das eingesetzte 2-Ethylanthrachinon zu 50 % zu 2-Ethylanthrahydrochinon umgesetzt (Bestimmung durch Gaschromatographie).
b) Gepackte Blasensäule: Es wurde insgesamt 3 h hydriert. Nach 3 h war das eingesetzte 2-Ethylanthrachinon zu 65 % zu 2-Ethylanthrahydrochinon umgesetzt (Bestimmung durch Gaschromatographie).

## Patentansprüche

1. Verfahren zur Suspensionshydrierung einer Anthrachinon-Verbindung oder eines Gemisches aus zwei oder mehr davon in einem Reaktor, der die Arbeitslösung enthält, in der mindestens ein Katalysator suspendiert ist, und der zusätzlich eine Wasserstoff enthaltende Gasphase enthält, **dadurch gekennzeichnet, daß** die Arbeitslösung und die Gasphase zumindest teilweise durch eine Vorrichtung mit Öffnungen oder Kanälen in dem Reaktor geführt werden, deren hydraulischer Durchmesser 0,5 bis 20 mm beträgt.

2. Verfahren nach Anspruch 1, **dadurch gekennzeichnet, daß** suspendierte Katalysatorpartikel mit einer mittleren Korngröße von 0,0001 bis 2 mm verwendet werden.

3. Verfahren nach Anspruch 1 oder 2, **dadurch gekennzeichnet, daß** als Vorrichtung mit Öffnungen oder Kanälen eine Schüttung, ein Gestrick, eine offenzellige Schaumstruktur oder ein Packungselement verwendet wird.

4. Verfahren nach einem der vorhergehenden Ansprüche, **dadurch gekennzeichnet, daß** die Arbeitslösung und die Gasphase zumindest teilweise durch Öffnungen oder Kanäle geführt werden, deren Wandmaterialien Oberflächenrauhigkeiten im Bereich des 0,1- bis 10fachen der mittleren Korngröße der suspendierten Katalysatorteilchen aufweisen.

5. Verfahren nach einem der vorhergehenden Ansprüche, **dadurch gekennzeichnet, daß** die Arbeitslösung und die Gasphase durch Öffnungen oder Kanäle mit metallischen Wandmaterialien geführt werden, deren Oberflächen einen Mittenrauhwert Rₐ nach DIN 4768/1 von 0,001 bis 0,01 mm aufweisen

6. Verfahren nach einem der vorhergehenden Ansprüche, **dadurch gekennzeichnet, daß** die Arbeitslösung mit einer Leerrohrgeschwindigkeit von 50 bis 300 m³/m²h, vorzugsweise von 150 bis 200 m³/m²h durch die Vorrichtung mit Öffnungen oder Kanälen geführt werden.

7. Verfahren nach einem der vorhergehenden Ansprüche, **dadurch gekennzeichnet, daß** der Reaktor eine, vorzugsweise vertikal stehend angeordnete, Blasensäule ist.

8. Verfahren nach einem der vorhergehenden Ansprüche, **dadurch gekennzeichnet, daß** der Reaktor ein heiz- oder kühlbarer Rohrbündelreaktor ist, wobei die Vorrichtung mit Öffnungen oder Kanälen in den einzelnen Rohren angebracht ist.

9. Verfahren nach einem der vorhergehenden Ansprüche, **dadurch gekennzeichnet, daß** der Reaktor ein Rührbehälter ist und die Vorrichtung mit den Öffnungen oder Kanälen in die Strombrecher und/oder die Rührorgane integriert ist.

10. Verfahren nach einem der vorhergehenden Ansprüche, wobei als Katalysator ein geträgerter Suspensionskatalysator verwendet wird.

11. Verfahren nach Anspruch 10, wobei der geträgerte Suspensionskatalysator mit einem Metall der VIII. Nebengruppe des Periodensystems beschichtet ist.

## Claims

1. A process for the suspension hydrogenation of an anthraquinone compound or a mixture of two or more thereof in a reactor in which there are present the working solution in which at least one catalyst is suspended and, in addition, a hydrogen-containing gas phase, wherein the working solution and the gas phase are, in the reactor, passed at least partly through a fitting having openings or channels whose hydraulic diameter is from 0.5 to 20 mm.

2. A process as claimed in claim 1, wherein suspended catalyst particles having a mean particle size of from 0.0001 to 2 mm are used.

3. A process as claimed in claim 1 or 2, wherein the fitting having openings or channels which is used is a bed, a knitted article, an open-celled foam or a packing element.

4. A process as claimed in any of the preceding claims, wherein the working solution and the gas phase are at least partly passed through openings or channels whose wall materials have surface roughnesses in the range from 0.1 to 10 times the mean particle size of the suspended catalyst particles.

5. A process as claimed in any of the preceding claims, wherein the working solution and the gas phase are passed through openings or channels having metallic wall materials whose surfaces have a mean roughness Rₐ in accordance with DIN 4768/1 of from 0.001 to 0.01 mm.

6. A process as claimed in any of the preceding claims, wherein the working solution is passed through the fitting having openings or channels at an empty tube velocity of from 50 to 300 m³/m²h, preferably from 150 to 200 m³/m²h.

7. A process as claimed in any of the preceding claims, wherein the reactor is a preferably upright bubble column.

8. A process as claimed in any of the preceding claims, wherein the reactor is a heatable or coolable multitube reactor, where the fitting . having openings or channels is installed in the individual tubes.

9. A process as claimed in any of the preceding claims, wherein the reactor is a stirred vessel and the fitting having the openings or channels is integrated into the baffle and/or the stirrers.

10. A process as claimed in any of the preceding claims, wherein the catalyst used is a supported suspension catalyst.

11. A process os claimed in claim 10, wherein the supported suspension catalyst is coated with a metal of transition group VIII of the Periodic Table.

## Revendications

1. Procédé pour l'hydrogénation en suspension d'un composé anthraquinone ou d'un mélange de deux ou de plusieurs de ceux-ci dans un réacteur, qui contient la solution de travail, dans lequel au moins un catalyseur est mis en suspension et qui contient en outre une phase gazeuse contenant de l'hydrogène, **caractérisé en ce que** la solution de travail et la phase gazeuse sont guidées dans le réacteur au moins partiellement à travers un dispositif présentant des ouvertures ou des canaux dont le diamètre hydraulique est de 0,5 à 20 mm.

2. Procédé selon la revendication 1, **caractérisé en ce qu'**on utilise des particules de catalyseur en suspension présentant une grosseur moyenne de granule de 0,0001 à 2 mm.

3. Procédé selon la revendication 1 ou 2, **caractérisé en ce qu'**on utilise comme dispositif présentant des ouvertures ou des canaux un matériau empilé, un tricot, une structure de mousse à cellules ouvertes ou un élément de garnissage.

4. Procédé selon l'une quelconque des revendications précédentes, **caractérisé en ce que** la solution de travail et la phase gazeuse sont guidées au moins partiellement à travers des ouvertures ou des canaux dont les matériaux formant les parois présentent des rugosités de surface représentant 0,1 à 10 fois la grosseur moyenne de granule des particules de catalyseur en suspension.

5. Procédé selon l'une quelconque des revendications précédentes, **caractérisé en ce que** la solution de travail et la phase gazeuse sont guidées à travers des ouvertures ou des canaux présentant des matériaux de paroi métalliques, dont la surface présente une valeur de rugosité moyenne Rₐ selon la norme DIN 4768/1 de 0,001 à 0,01 mm.

6. Procédé selon l'une quelconque des revendications précédentes, **caractérisé en ce que** la solution de travail est guidée à travers le dispositif présentant des ouvertures ou des canaux à une vitesse dans un tuyau vide de 50 à 300 m³/m²h, de préférence de 150 à 200 m³/m²h.

7. Procédé selon l'une quelconque des revendications précédentes, **caractérisé en ce que** le réacteur est une colonne à bulles, de préférence disposée verticalement.

8. Procédé selon l'une quelconque des revendications précédentes, **caractérisé en ce que** le réacteur est un réacteur à faisceau tubulaire pouvant être chauffé ou refroidi, le dispositif présentant des ouvertures ou des canaux étant disposé dans les différents tubes.

9. Procédé selon l'une quelconque des revendications précédentes, **caractérisé en ce que** le réacteur est un conteneur agité et le dispositif présentant les ouvertures ou les canaux est intégré dans les chicanes et/ou les organes d'agitation.

10. Procédé selon l'une quelconque des revendications précédentes, **caractérisé en ce qu'**on utilise comme catalyseur un catalyseur en suspension supporté.

11. Procédé selon la revendication 10, le catalyseur en suspension supporté étant revêtu par un métal du VIIIème groupe secondaire du système périodique.
